(19) **Europäisches Patentamt / European Patent Office / Office européen des brevets**

(11) **EP 1 839 647 B1**

(12) **EUROPEAN PATENT SPECIFICATION**

(45) Date of publication and mention
of the grant of the patent:
**01.02.2012 Bulletin 2012/05**

(21) Application number: **05814307.4**

(22) Date of filing: **01.12.2005**

(51) Int Cl.:
*A61K 8/92* (2006.01)   *A61K 8/89* (2006.01)
*A61Q 19/00* (2006.01)

(86) International application number:
**PCT/JP2005/022514**

(87) International publication number:
**WO 2006/059798 (08.06.2006 Gazette 2006/23)**

(54) **OILY SKIN COMPOSITION FOR EXTERNAL USE FOR ACCELERATING PERCUTANEOUS ABSORPTION**

ÖLIGE HAUTZUSAMMENSETZUNG ZUR TOPISCHEN ANWENDUNG ZUR BESCHLEUNIGUNG DER PERKUTANEN RESORPTION

PRÉPARATION CUTANÉE HUILEUSE À USAGE EXTERNE POUR L'ACCÉLÉRATION DE L'ABSORPTION PERCUTANÉE

(84) Designated Contracting States:
**DE ES FR GB IT**

(30) Priority: **02.12.2004 JP 2004349575**

(43) Date of publication of application:
**03.10.2007 Bulletin 2007/40**

(73) Proprietor: **SHISEIDO COMPANY, LTD.**
**Tokyo 104-8010 (JP)**

(72) Inventors:
• **YOSHIDA, Mari,**
**Shiseido Research Center**
**Yokohama-shi, Kanagawa 224-8558 (JP)**
• **TAKAHASHI, Akiko,**
**Shiseido Research Center**
**Yokohama-shi, Kanagawa 224-8558 (JP)**
• **OKAMOTO, Tohru,**
**Shiseido Research Center**
**Yokohama-shi, Kanagawa 224-8558 (JP)**

(74) Representative: **Winkler, Andreas Fritz Ernst et al**
**Forrester & Boehmert**
**Pettenkoferstrasse 20-22**
**80336 München (DE)**

(56) References cited:
WO-A2-02/060502     JP-A- 07 196 480
JP-A- 09 194 323     JP-A- 09 263 525
JP-A- 2000 063 254     JP-A- 2002 322 027
JP-A- 2003 026 608     JP-A- 2005 097 191
JP-A- 2005 170 842     US-A- 5 326 566
US-A1- 2003 228 335     US-A1- 2004 197 281

• DATABASE WPI Week 200364 Thomson Scientific, London, GB; AN 2003-673547 XP002573493 & JP 2003 026608 A (NIPPON OILS & FATS CO LTD) 29 January 2003 (2003-01-29)

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

This invention relates to an oil based composition for external use on skin for enhancing percutaneous absorption, the oil based composition being adapted for use such that, after a preparation for external use on skin, which preparation contains a water-soluble agent, has been applied onto skin, the oil based composition may be applied onto the preparation for external use on skin, which preparation has been applied onto the skin, in order to enhance percutaneous absorption of the water-soluble agent contained in the preparation for external use on skin. This invention also relates to a method of enhancing percutaneous absorption of a water-soluble agent by use of the oil based composition.

Background Art

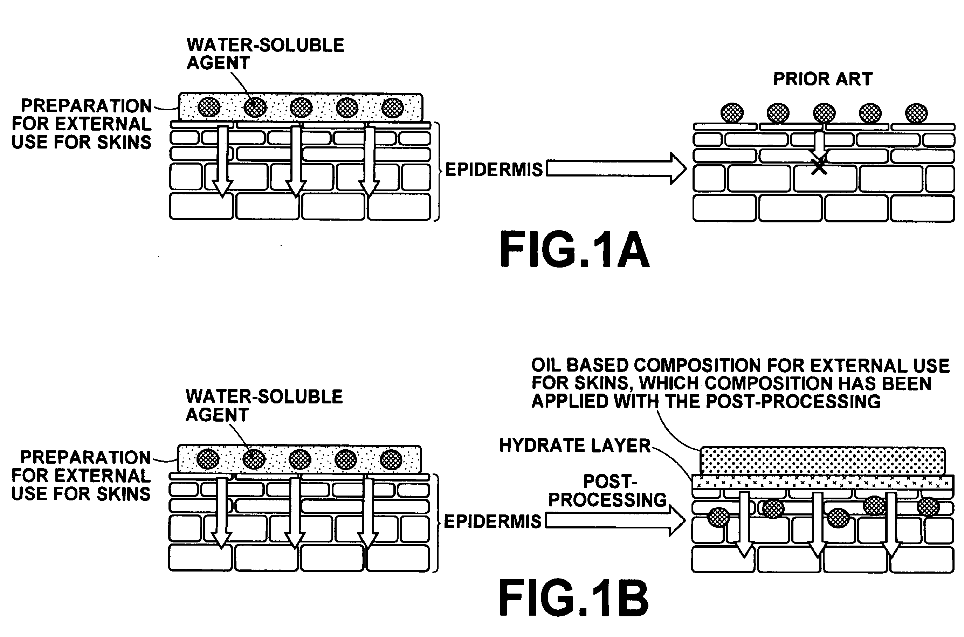

Preparations for external use on skin, such as ointments, milky lotions, skin creams, lotions, and gels, contain various kinds of water-soluble agents, such as anti-oxidants, hemokinesis enhancing agents, whitening agents, moisturizer, and vitamins. At the time at which the preparation for external use on skin has been applied onto skin, the water-soluble agent contained in the preparation for external use on skin is capable of permeating through the skin as long as the water-soluble agent has been dissolved in water. However, in cases where water contained in a base is vaporized and lost, and the water-soluble agent is crystallized on the surface of the skin, the permeation of the water-soluble agent through the skin is suppressed. Therefore, the problems have heretofore been encountered in that small absorption of the water-soluble agent into the skin occurs, and in that sufficient effects of the water-soluble agent are not capable of being obtained. Figure 1A is an explanatory view showing how permeation of a water-soluble agent, which is contained in a preparation for external use on skin, through a skin is suppressed in the cases of a conventional technique.

Heretofore, various attempts have been conducted to enhance the permeability of the water-soluble agents, which are contained in the preparations for external use on skin, through the skins. For example, an attempt has heretofore been made to form a micell (as described in, for example, U.S. Patent No. 5,747,066. Also, attempts have heretofore been made to blend specific constituents capable of enhancing the permeability of the water-soluble agents (as described in, for example, Japanese Unexamined Patent Publication Nos. 9(1997)-157129, 5(1993)-229927, and 2000-178125). However, the attempted techniques are not always capable of being applied to every preparation for external use on skin. Also, the attempted techniques require the formation of preparations and are therefore not always capable of being utilized easily. Further, in cases where the water-soluble agents are formed into preparations by use of oil-based composition, and the like, the problems occur in that the usability of the obtained preparations is not capable of being kept good, and in that, since the water-soluble agents do not come into direct contact with the skins, the percutaneous absorption of the water-soluble agents themselves is not capable of being enhanced sufficiently.

Furthermore, such that the usability of oily base materials, such as petrolatums, which are used in cosmetic preparations and other preparations for external use on skin for the purposes of skinprotection and enhancement of occlusivity, may be improved, attempts have heretofore been made to blend various kinds of particles.

A preparation for external use on skin, containing: (A) white petrolatum, (B) powder for cosmetic use, such as particles of anhydrous silicic acid, hydrous silicic acid, calciumsilicate, titanium dioxide, and nylon, (C) a polymer containing a phosphoryl choline-like group, and (D) an antiphlogistic or a microbicide is disclosed in, for example, Japanese Unexamined Patent Publication No. 2003-026608. It is described that the blending of the particles within the preparation for external use on skin enables suppression of a sticky feeling and shininess of the skin when the preparation is applied thereon. It is described that the blending of the particles enables suppression of a sticky feeling and a shininess of skin, after the coating of the preparation for external use on skin.

A paste preparation containing an oily base material blended with fine particles of polyacrylic acid salts and gluten particles, or further blended with gelatin particles is disclosed in Japanese Unexamined Patent Publication No. 6(1994)-316516. It is described that the proposed paste preparation exhibits good long lasting property and good stability.

Further, a solid particle cosmetic preparation containing spherical particles, which have a mean particle diameter falling within the range of 3.0μm to 20.0μm, and a hydrocarbon wax is disclosed in Japanese Unexamined Patent Publication No. 9(1997)-012429. It is described that extensibility and smoothness are capable of being improved by the blending of the spherical particles.

US 2004/197281 A1 describes an antiperspirant stick composition for application to human skin, which comprises from about 5 wt% to about 35 wt% of an antiperspirant active; from about 5 wt% to about 35 wt% of a structurant; from about 20 wt% to about 80 wt% of a volatile fluid; and 10 wt% or less of a non-volatile organic fluid, wherein the stick has a hardness of at least about 600 gram force and an adhesion value of at least 33%.

WO 02/060502 A2 describes the inclusion of non-aqueous compositions that contain anionic polymers on the bodyfacing materials of disposable absorbent articles.

US 5,326,566 describes a composition for enhancing and/or controlling a epidermal, dermal and transdermal penetration of topically applied pharmacologically active agents by use of dibutyl adipate, or a mixture of dibutyl adipate and isopropyl myristate.

US 2003/228335 A1 teaches that the sensory characteristics of anhydrous semisolid lipids can be improved with the addition of a combination of amphiphobic micro or sub-micro particles of polymerized perhalogenoalkenes and at least one amphophilic lipid containing at least two hydroxyl groups.

In view of the above circumstances, the object of the present invention is to provide an oil based composition for external use on skin for enhancing percutaneous absorption, which composition need not be formed into a particular preparation, which composition is capable of enhancing percutaneous absorption of various kinds of water-soluble agents contained in preparations for external use on skin, and which composition exhibits good usability and good stability. Another object of the present invention is to provide a method of enhancing percutaneous absorption of a water-soluble agent by use of the oil based composition for external use on skin for enhancing percutaneous absorption.

Disclosure of the Invention

The inventors have found that, with post-treatment wherein, after a preparation for external use on skin, which preparation contains a water-soluble agent, has been applied onto skin, an oil based composition for external use on skin, which oil based composition has occlusivity as high as at least 50%, is applied from above the preparation for external use on skin, which preparation has been applied onto the skin, water is capable of retained on the surface of the skin in a state, in which the water-soluble agent is in contact with the surface of the skin, the dissolved state of the water-soluble agent on the surface of the skin is capable of being kept for a long period of time, and the percutaneous absorption of the water-soluble agent is capable of being enhanced markedly. Figure 1B is an explanatory view showing how percutaneous absorption of a water-soluble agent is enhanced by post-processing with an oil based composition for external use on skin in accordance with the present invention.

However, the conventional oil-based composition having high occlusivity, such as a petrolatum, exhibits a low spreadability. Also, at the time at which the conventional oily base material is applied onto the skin, the conventional oilybase material gives a sticky feeling and a shininess of skin. The usability of the conventional oily base material is thus markedly bad. As described above, such that the usability of the petrolatum, or the like, may be improved, various attempts have heretofore been made to blend various kinds of particles. (Reference may be made to, for example, Japanese Unexamined Patent Publication Nos. 2003-026608, 6(1994)-316516, and 9(1997)-012429.) However, with the conventional compositions, wherein the particles are blended, paths allowing the passage of water are formed through the oil based system, and the occlusivity becomes low. Therefore, with the conventional compositions, in cases where the conventional compositions are used such that, after the preparation for external use on skin, which preparation contains the water-soluble agent, has been applied onto the skin, each of the conventional compositions is applied from above the preparation for external use on skin, which preparation has been applied onto the skin, the effects of enhancing the percutaneous absorption of the water-soluble agent are not capable of being obtained. The inventors have found that, in cases where 50% by mass to 95% by mass of an oil constituent, which contains 10% by mass to 100% by mass of a solid or semisolid oil constituent, and 5% by mass to 50% by mass of particles are blended together, the usability is capable of being improved markedly, such that the occlusivity of the oil based composition for external use may not be affected adversely. The present invention is based upon the findings described above.

Specifically, the present invention provides an oil based composition for external use on skin for enhancing percutaneous absorption of a water-soluble agent as defined in claim 1.

The present invention also provides a cosmetic method as defined in claim 3.

Each of the method of enhancing percutaneous absorption of a water-soluble agent in accordance with the present invention and the beauty method in accordance with the present invention may be modified such that the method further comprises the step of performing an iontophoresis on the skin at a stage between when the preparation for external use on skin, which preparation contains the water-soluble agent, or the cosmetic preparation, which contains the water-soluble agent, has been applied onto the skin and when the oil based composition for external use on skin is applied onto the preparation for external use on skin, which preparation has been applied onto the skin, or onto the cosmetic preparation, which has been applied onto the skin. With the combination of the post-treatment, which is performed by use of the oil based composition for external use on skin in accordance with the present invention, and the iontophoresis, the percutaneous absorption of the water-soluble agent is capable of being enhanced more efficiently.

The term "occlusivity" as used herein means the value calculated with the formula shown below and in accordance with a transepidermal water loss (TEWL), which is measured with a water loss meter at a stage one hour after a sample has been applied (at a rate of 2.5mg/cm$^2$) to an medical site of a human forearm.

```
Occlusivity (%)

= (1-TEWL (with sample) / TEWL (without sample)) × 100
```

In the present invention, the particles shouldpreferably contain elastic particles or spherical particles. The elastic particles and the spherical particles have good effects of improving the sticky feeling. Also, in cases where the spherical particles are blended with the oil based composition in accordance with the present invention, wrinkles of the skin are capable of being blurred and rendered imperceptible by virtue of the light scattering effects of the spherical particles contained in the oil based composition having been applied onto the skin. In cases where the elastic particles are blended with the oil based composition in accordance with the present invention, a good usability without a powdery feeling is capable of being obtained. Particularly, in cases where elastic silicone type particles or spherical silicone type particles are blended with the oil based composition in accordance with the present invention, spreadability on skin of the oil based composition in accordance with the present invention are capable of being performed smoothly, no powdery feeling is given, and a particularly good usability is capable of being obtained.

From the view point of the occlusivity, the oil constituent should preferably contain a solid or semisolid non-polar hydrocarbon oil constituent in a proportion of at least 20% by mass with respect to a total quantity of the oil constituent. In cases where the non-polar hydrocarbon oil constituent is blended at a high concentration, the occlusivity are capable of being enhanced even further.

Also, from the view point of the usability, the oil constituent should preferably contain a volatile oil constituent in a proportion falling within the range of 5% by mass to 50% by mass with respect to a total quantity of the oil constituent. In cases where the volatile oil constituent is blended, spreadability on skin are capable of being performed more easily, and the sticky feeling of the coated composition is capable of being improved even further.

The oil based composition for external use on skin in accordance with the present invention has the occlusivity of at least 50%. Therefore, in cases where the oil based composition for external use on skin in accordance with the present invention is applied onto the preparation for external use on skin, which preparation contains the water-soluble agent and has been applied onto the skin, the surface of the layer of the preparation for external use on skin is capable of being occluded in the state, in which the water-soluble agent contained in the preparation for external use on skin is in contact with the surface of the skin. Water coming from the skin is thus capable of being kept at the surface of the skin, and the dissolved state of the water-soluble agent is capable of being kept at the surface of the skin. As a result, the permeation of the water-soluble agent is capable of being continued. Therefore, with the oil based composition for external use on skin in accordance with the present invention, without using special formulation, and the percutaneous absorption of various water-soluble agents contained in the conventional preparations for external use on skin is capable of being enhanced easily. Also, the oil based composition for external use on skin in accordance with the present invention contains 50% by mass to 95% by mass of the oil constituent, which contains 10% by mass to 100% by mass of the solid or semisolid oil constituent, and 5% by mass to 50% by mass of the particles. Therefore, in cases where the oil based composition for external use on skin in accordance with the present invention is applied onto the skin, the oil based composition does not give the sticky feeling and the shininess of skin and exhibits good characteristics of occluding the skin. Also, the oil based composition in accordance with the present invention has a markedly good spreadability. Accordingly, the oil based composition in accordance with the present invention is capable of having sufficient occlusivity in cases where a small quantity of the oil based composition is used.

Brief Description of the Drawings

Figure 1A is an explanatory view showing how permeation of a water-soluble agent, which is contained in a preparation for external use on skin, penetration into skin of water soluble agent is suppressed in the cases of a conventional technique, and

Figure 1B is an explanatory view showing how percutaneous absorption of a water-soluble agent is enhanced by a method in accordance with the present invention.

Best Mode for Carrying Out the Invention

Each of the oil based composition for external use on skin in accordance with the present invention and the oil based composition for external use on skin, which oil based composition is employed in the method in accordance with the present invention, contains the oil constituent, which contains the solid or semisolid oil constituent, and the particles.

In the present invention, the blending proportion of the oil constituent contained in the oil based composition for external use on skin falls within the range of 50% by mass to 95% by mass with respect to the total quantity of the composition. The blending proportion of the oil constituent contained in the oil based composition for external use on skin should preferably fall within the range of 60% bymass to 90% bymass with respect to the total quantity of the composition, and should more preferably fall within the range of 70% by mass to 85% by mass with respect to the total quantity of the composition. If the blending proportion of the oil constituent is lower than 50% by mass, high occlusivity will not be capable of being obtained. If the blending proportion of the oil constituent is higher than 95% by mass, the usability will become bad.

The solid or semisolid oil constituent employed in the present invention may be selected from a wide variety of oil constituents, which are solid or semisolid at normal temperatures (25°C). Examples of the solid or semisolid oil constituents include solid paraffin, micro-crystalline wax, ceresine, bees wax, bareco wax, polyethylene wax, silicon wax, behenyl alcohol, stearyl alcohol, cetyl alcohol, Batyl alcohol, carnauba wax, bees wax, candelilla wax, jojoba wax, lanolin, shellac wax, whale wax, Japanese wax, myristic acid, palmitic acid, stearic acid, behenic acid, 12-hydroxystearicacid, cacao butter, hardened castor bean oil, hardened oil, hydrogenated palm oil, palm oil, hardened coconut oil, polyethylene powder, petrolatum, various kinds of hydrogenated animal fats and oils, variouskindsofhydrogenated vegetable fats and oils, and fatty acid monocarboxylic acid lanolin alcohol esters. In the present invention, the solid or semisolid oil constituent acts to efficiently prevent water from being vaporized and lost from the skin, to retain water at the surface of the skin, and to keep the dissolved state of the water-soluble agent. Therefore, the solid or semisolid oil constituent employed should preferably have high occlusivity. The solid or semisolid oil constituent should preferably be the solid or semisolid non-polar hydrocarbon oil, such as micro-crystalline wax, polyethylene wax, or petrolatum.

In the present invention, the blending proportion of the solid or semisolid oil constituent falls within the range of 10% by mass to 100% bymass with respect to the total quantity of the oil constituent. If the blending proportion of the solid or semisolid oil constituent is lower than 10% by mass, high occlusivity will not be capable of being obtained. A preferable blending proportion of the solid or semisolid oil constituent is not limited and may vary in accordance with the kind of the oil constituent employed and the oil constituent combination. For example, the blending proportion of the solid or semisolid oil constituent should preferably fall within the range of 20% by mass to 95% by mass with respect to the total quantity of the oil constituent, and should more preferably fall within the range of 30% by mass to 90% by mass with respect to the total quantity of the oil constituent. Particularly, the solid or semisolid non-polar hydrocarbon oil constituent should preferably be contained in a proportion of at least 20% with respect to the total quantity of the oil constituent, should more preferably be contained in a proportion of at least 30% with respect to the total quantity of the oil constituent, and should most preferably be contained in a proportion of at least 40% with respect to the total quantity of the oil constituent. In cases where the solid or semisolidnon-polar hydrocarbon oil constituent is blended at a high blending proportion, particularly high occlusivity and high-temperature stability are capable of being obtained.

In the present invention, from the view point of the usability, the oil constituent should preferably contain the volatile oil constituent. In cases where the volatile oil constituent is blended, the coating and extension and the sticky feeling of the coating composition are capable of being improved even further.

The term "volatile oil constituent" as used herein means the oilconstituent, which has the volatility at the room temperature (25°C) . In the present invention, the volatile oil constituent may be selected from a wide variety of oil constituents, with which the purposes of the present invention are capable of being accomplished. Examples of the volatile oil constituents include isoparaffin type hydrocarbon oils having a low boiling temperature (a boiling temperature of at most 260°C at the normal pressure) and silicone oils having a low boiling temperature.

Specifically, the isoparaffin type hydrocarbon oils having a low boiling temperature are commercially available under the trade names of Isopar A, Isopar C, Isopar E, Isopar G, Isopar H, Isopar K, Isopar L, and Isopar M (supplied by Exxon Co.) ; Shellsole 71 (supplied by Shell Co.); Soltrol 100, Soltrol 130, and Soltrol 220 (supplied by Phillip Co.).

Examples of the preferable silicone oils having a low boiling temperature include hexamethylcyclotrisiloxane; octamethyltetracyclosiloxane (e.g., Execol D-4, supplied by Shin-Etsu Silicone Co.); SH244, SH344 (supplied by Dow Corning Toray Silicone Co., Ltd.); decamethylcyclopentasiloxane (e.g., Execol D-5, supplied by Shin-Etsu Silicone Co.); SH245, DC345 (supplied by Dow Corning Toray Silicone Co., Ltd.); dodecamethylcyclohexasiloxane (e.g., DC246, supplied by Dow Corning Toray Silicone Co., Ltd.); and tetradecamethylcycloheptasiloxane. Particularly, for the advantages of having good usability and yielding high occlusivity, decamethylcyclopentasiloxane should preferably be blended as the volatile oil constituent.

In the present invention, one kindof the volatile oil constituent may be used alone. Alternatively, two or more kinds of the volatile oil constituents may be used in combination. The blending proportion of the volatile oil constituent should preferably fall within the range of 5% by mass to 50% by mass with respect to the total quantity of the oil constituent. The blending proportion of the volatile oil constituent should more preferably fall within the range of 10% by mass to 40% by mass with respect to the total quantity of the oil constituent, and should most preferably fall within the range of 15% by mass to 30% by mass with respect to the total quantity of the oil constituent.

Also, a liquid oil constituent, which is liquid at the room temperature (25°C), may be blended, such that the purposes of the present invention may be accomplished. Particularly, since the solid oil constituent itself is markedly hard and exhibits a low usability, the liquid oil constituent should preferably be contained together with the solid oil constituent. Examples of the liquid oil constituents include non-polar hydrocarbon oils, such as liquid paraffin, and squalane; fats and oils, such as olive oil, macadamia nut oil, and jojoba oil; higher fatty acids, such as oleic acid, tall oil fatty acid, and isostearic acid; higher alcohols, such as lauryl alcohol, oleyl alcohol, isostearyl alcohol, and octyldodecanol; esters, such as isocetyl isostearate, myristyl myristate, and isopropyl palmitate; chain polysiloxanes, such as dimethylpolysiloxane, methylphenylpolysiloxane, and methylhydrogenpolysiloxane; ultraviolet light absorbers, such as benzophenone derivatives; and perfumes. Particularly, from the view point of the occlusivity, the non-polar hydrocarbon type of liquid oil, such as liquid paraffin or squalane, should preferably be blended. One kind of the liquid oil constituent enumerated above may be used alone. Alternatively, two to more kinds of the liquid oil constituents enumerated above may be used in combination. Also, the blending proportion of the liquid oil constituent is not limited and may vary in accordance with the kind of the oil constituent employed and the oil constituent combination. The blending proportion of the liquid oil constituent should preferably fall within the range of 10% by mass to 90% by mass with respect to the total quantity of the oil constituent. The blending proportion of the liquid oil constituent should more preferably fall within the range of 20% by mass to 80% by mass with respect to the total quantity of the oil constituent, and should most preferably fall within the range of 30% by mass to 70% by mass with respect to the total quantity of the oil constituent.

The particles employed in the present invention may be selected from a wide variety of kinds of particles, with which the purposes of the present invention are capable of being accomplished. Particularly, particles of extender pigments are preferable. Examples of the preferable particles, which may be employed in the present invention, include plate-shaped particles, such as talc particles, kaolin particles, and sericite particles; and spherical particles, such as polyethylene particles, polymethyl methacrylate particles, polystyrene particles, nylon particles, silica particles, silicone resin particles, silicone rubber particles, silicone resin-coated silicone rubber particles, and polyurethane particles. The spherical particles have the light scattering effects. Therefore, in cases where the spherical particles are blended, wrinkles of the skin are capable of being blurred and rendered imperceptible. Also, in cases where the elastic particles, such as the silicone rubber particles, silicone resin-coated silicone rubber particles, and polyurethane particles, are blended, a feeling free from a powdery feeling is capable of being obtained. Therefore, the elastic particles described above should preferably be blended. Particularly, the elastic silicone type particles or the spherical silicone type particles should preferably be blended. Examples of the elastic silicone type particles or the spherical silicone type particles include spherical silicone rubber particles, spherical silicone resin particles, silicone resin-coated silicone rubber particles, and zinc oxide-coated, silicone resin-coated silicone rubber particles. It is more preferable to blend the elastic silicone type particles or the spherical silicone type particles, which have a mean particle diameter falling within the range of $1\mu$m to $50\mu$m. In such cases, the coating and the extension become smooth, a powdery feeling is capable of being eliminated, and markedly good usability is capable of being obtained.

Further, various kinds of the extender pigments may be coated with particles having a refractive index of at least 1. 6, such as barium sulfate (refractive index: 1.64), zinc oxide (refractive index: 2.0), and titanium oxide (rutile type) (refractive index: 2.7), or may be formed as composite particles by use of the aforesaid particles having a refractive index of at least 1. 6. Alternatively, part of the particles may be subjected to simple mixing with the aforesaid particles having a refractive index of at least 1.6. In this manner, it is possible to obtain high color nonuniformity concealing effects.

In cases where part of the particles are subjected to the simple mixing with the aforesaid particles having a refractive index of at least 1.6, the aforesaid particles having a refractive index of at least 1. 6 should preferably be blended in a proportion of approximately 0.5% by mass to approximately 3% by mass in the oil based composition for external use on skin in accordance with the present invention. If the proportion of the aforesaid particles having a refractive index of at least 1.6 in the oil based composition for external use on skin in accordance with the present invention is higher than 3% by mass, at the time at which the oil based composition is coated onto the skin, the color of the skin will become markedly white and an unnatural feeling will be given.

In cases where the particles coated with the aforesaid particles having a refractive index of at least 1.6 or the composite particles formed by use of the aforesaid particles having a refractive index of at least 1.6 are blended, the particles coated with the aforesaid particles having a refractive index of at least 1.6 or the composite particles formed by use of the aforesaid particles having a refractive index of at least 1.6 may be blended in a proportion of approximately 5% by mass to approximately 30% by mass in the oil based composition for external use on skin in accordance with the present invention. In such cases, it is possible to obtain high color nonuniformity concealing effects. In cases where the particles coated with the aforesaid particles having a refractive index of at least 1.6 or the composite particles formed by use of the aforesaid particles having a refractive index of at least 1. 6 are blended, it is possible to obtain the color nonuniformity concealing effects higher than in cases where part of the particles are subjected to the simple mixing with the aforesaid particles having a refractive index of at least 1.6. Also, in cases where the particles coated with the aforesaid particles having a refractive index of at least 1.6 or the composite particles formed by use of the aforesaid particles having a refractive index of at least 1.6 are blended, at the time at which the oil based composition for external use on skin is coated onto the skin, it is possible to obtain a skin feeling more beautiful than in cases where part of the particles are subjected to the simple mixing with the aforesaid particles having a refractive index of at least 1.6. Therefore, it is more preferable to blend the particles coated with the aforesaid particles having a refractive index of at least 1.6 or the composite particles formed by use of the aforesaid particles having a refractive index of at least 1.6.

In the oil based composition for external use on skin in accordance with the present invention, one kind of the particles may be blended. Alternatively, two or more kinds of the particles may be blended. The blending proportion of the particles in the oil based composition for external use on skin in accordance with the present invention should fall within the range of 5% by mass to 50% by mass with respect to the total quantity of the composition. The blending proportion of the particles in the oil based composition for external use on skin in accordance with the present invention should preferably fall within the range of 10% by mass to 40% by mass with respect to the total quantity of the composition, and should more preferably fall within the range of 15% by mass to 30% by mass with respect to the total quantity of the composition. If the blending proportion of the particles in the oil based composition for external use on skin in accordance with the present invention is lower than 5% by mass with respect to the total quantity of the composition, the shininess of skin with the oil constituent, the sticky feeling with the oil constituent, and the like, will not capable of being improved sufficiently. If the blending proportion of the particles in the oil based composition for external use on skin in accordance with the present invention is higher than 50% by mass with respect to the total quantity of the composition, the occlusivity will become bad.

In the present invention, the oil based composition for external use on skin should have the occlusivity of at least 50%. The oil based composition for external use on skin in accordance with the present invention should preferably have the occlusivity higher than 50%, for example, the occlusivity of at least 60%. The oil based composition for external use on skin in accordance with the present invention should more preferably have the occlusivity of at least 70%, and should most preferably have the occlusivity of at least 80%. The occlusivity may be calculated in the manner described below. Specifically, a sample is applied (at a rate of 2.5mg/cm$^2$) to an medical site of a human forearm. At a stage one hour after the sample has been applied (at a rate of 2.5mg/cm$^2$) to the internal site of the human forearm, the transepidermal water loss (TEWL) is measured with a water loss meter, such as Tewameter TM210 (supplied by Courage+Khazaka Co.), MEECO (supplied by Meeco Co., Warrington, PA, USA), Vapometer, or TEWA meter (supplied by Delfin Technologies Ltd., Kuopio, Finland). Thereafter, the occlusivity are calculated with the formula shown below and in accordance with the transepidermal water loss (TEWL) having thus been measured.

```
Occlusivity (%)
= (1-TEWL (with sample) / TEWL (without sample)) × 100
```

The oil based composition for external use on skin in accordance with the present invention may embrace cosmetic preparations, pharmaceutical preparations, quasi-drugs, and the like. Also, the oil based composition for external use on skin in accordance with the present invention may take on a wide variety of preparation forms, such as an ointment type, a paste type, and a skin cream type.

When necessary, besides the essential constituents described above, the oil based composition for external use on skin in accordance with the present invention may also contain other arbitrary constituents, which are ordinarily used in compositions for external use on skin, such as the cosmetic preparations and the pharmaceutical preparations, within a range such that the effects of the present invention may not be affected adversely. Examples of the other arbitrary constituents, which are ordinarily used in compositions for external use on skin, such as the cosmetic preparations and the pharmaceutical preparations, include a moisture retaining agent, a surface active agent, an ultraviolet light absorber, a perfume, an anti-oxidant, an antiseptic agent, a mildewproofing agent, an extender pigment, a coloring material (such as a coloring pigment), and a pH regulating agent. From the view point of the occlusivity, the other arbitrary constituents described above should preferably be substantially free from water and water-soluble constituents. However, the other arbitrary constituents described above may take on the form of W/O type emulsions containing a small amount of water, such that the purposes of the present invention are capable of being accomplished. By way of example, the proportion of water and water-soluble constituents contained in the oil based composition for external use on skin may be at most 10% by mass. The proportion of water and water-soluble constituents contained in the oil based composition for external use on skin should preferably be at most 5% by mass, and should more preferably be at most 1% by mass.

In the present invention, the preparation for external use on skin, which preparation contains the water-soluble agent, may embrace cosmetic preparations, pharmaceutical preparations, quasi-drugs, and the like. Also, besides the water-soluble agent, the preparation for external use on skin, which preparation contains the water-soluble agent, may also contain arbitrary constituents, which are ordinarily contained in compositions for external use on skin. Further, the preparation for external use on skin, which preparation contains the water-soluble agent, may take on a wide variety of preparation forms, such as a solution type, an emulsion type, a skin cream type, a lotion type, and a gel type.

The term "water-soluble agent" as used herein means an arbitrary active constituent, which is soluble in water. The water-soluble agent is not limited to a specific agent and may be, for example, a whitening agent, an antiphlogistic, an antimicrobial agent, a hormone agent, a vitamin agent, an enzyme, an anti-oxidant, a hemokinesis enhancing agent, an amino acid, a hair growth agent, an animal extract, or a vegetable extract.

Examples of the whitening agents include hydroquinone derivatives, such as hydroquinone-α-D-glucose, hydroquinone-β-D-glucose (referred to also as arbutin), hydroquinone-α-L-glucose, hydroquinone-β-L-glucose, hydroquinone-α-D-galactose, hydroquinone-β-D-galactose, hydroquinone-α-L-galactose, and hydroquinone-β-L-galactose; kojic acid and derivatives of kojic acid; L-ascorbic acid and derivatives of L-ascorbic acid, such as L-ascorbic acid monoesters (e.g., an L-ascorbic acid monophosphoric acid ester and an L-ascorbic acid 2-sulfuric acid ester), L-ascorbic acid glucosides (e.g., L-ascorbic acid2-glucoside), and salts of the aforesaidL-ascorbic acidmonoesters or the aforesaid L-ascorbic acid glucosides; tranexamic acid and derivatives of tranexamic acid, such as tranexamic acid, dimers of tranexamic acid [e.g., hydrochloric acid trans-4-(trans-aminomethylcyclohexanecarbonyl)aminomethylcyclohex anecarboxylic acid], esters of tranexamic acid and hydroquinone [e.g., a trans-4-aminomethylcyclohexanecarboxylic acid 4'-hydroxyphenyl ester], esters of tranexamic acid and gentisic acid [e.g., 2-(trans-4-aminomethylcyclohexylcarbonyloxy)-5-hydroxybenzoic acid and salts thereof], amides of tranexamic acid [e.g., trans-4-aminomethylcyclohexanecarboxylic acid methyl amide and salts thereof, trans-4-(p-methoxybenzoyl)aminomethylcyclohexanecarboxylic acid and salts thereof, and trans-4-guanidinomethylcyclohexanecarboxylic acid and salts thereof]; ellagic acid and derivatives of ellagic acid; salicylic acid and derivatives of salicylic acid, such as salicylic acid, 3-methoxysalicylic acid and salts thereof, 4-methoxysalicylic acid and salts thereof, and 5-methoxysalicylic acid and salts thereof; resorcinol derivatives, such as resorcin, alkyl resorcinols, e.g., 4-n-butylresorcinol, and salts thereof; and vegetable extracts having whitening effects.

Examples of the antiphlogistics include glycyrrhizic acid salts (e.g., a glycyrrhizic acid dipotassium salt and a glycyrrhizic acid ammonium salt), and allantoin.

Examples of the antimicrobial agents include resorcin, sulfur, salicylic acid, zinc pyrithione, photosensitizer No. 101, photosensitizer No. 102, octopirox, and hinokitiol.

Examples of the hormone agents include oxytocin, corticotropin, vasopressin, secretin, gastrin, and calcitonin.

Examples of vitamin agents include vitamin $B_6$, vitamin $B_6$ derivatives, such as vitamin $B_6$ hydrochloride, vitamin $B_2$, vitamin $B_{12}$, nicotinic acid, nicotinic acid derivatives, such as nicotinic acid amide, and a pantothenyl ethyl ether.

Examples of the enzymes include trypsin, lysozyme chloride, chymotrypsin, semialkali endopeptidase, serrapeptase, lipase, and hyaluronidase.

Examples of the anti-oxidants include thiotaurine, glutathione, catechin, albumin, ferritin, and metallothionein.

Examples of the hemokinesis enhancing agents include acetylcholine derivatives, cepharanthine, and carpronium chloride.

Examples of the amino acids include serine, methionine, and tryptophan.

Examples of the hair growth agents include hemokinesis enhancing agents, such as a Swertia herb extract, acetyl choline derivatives, cepharanthine, and carpronium chloride; local irritants, such as capsicum tincture, a cantharis extract, and nonylic acid vanylamide; anti-seborrhea agents, such as pyridoxine or derivatives thereof; antimicrobial agents, such as benzalkonium chloride, isopropylmethyl phenol, zinc pyrithione, photosensitizer No. 101, photosensitizer No. 102, octopirox, and hinokitiol; metabolism activators, such as photosensitizer No. 301, a placenta extract, and biotin; amino acids, such as serine, methionine, and tryptophan; and vitamin agents, such as vitamin $B_2$, vitamin $B_{12}$, pantothenic acid or derivatives of pantothenic acid.

Of the animal extracts and the vegetable extracts, examples of the vegetable extracts include a tea extract, an extract of *rosa roxburghii,* an extract of Scutellaria root, an extract of *Houttuynia cordata Thunb.,* anextractofPhellodendronBark, anextractofMelilotus, an extract of *Lamium album* L. var. *barbatum* (Sieb. et Zucc.) Franch. et Savat., a Glycyrrhiza extract, an extract of *Paeonia lactiflora* Pall., an extract of common soapwort, an extract of loofah, an extract of cinchona, an extract of *Saxifraga stolonifera* Meerb., an extract of *Sophora flavescens* Aiton, an extract of *Nuphar japonicum* DC., an extract of *Foeniculum vulgare* Mill., an extract of *Primula sieboldii* E. Morren, an extract of rose, an extract of *Rehmannia. glutinosa* Libosch. var. *purpurea* Makino, an extract of lemon, an extract of Lithospermum root, an extract of Aloe, an extract of root of *Acorus calamus* L. var. *asiaticus* Pers., an extract of *Eucalyptus globulus* Labill., an extract of *Equisetum arbense* L., an extract of *Salvia officinalis* L., an extract of *Thymus vulgaris* L. (Common Thyme), extracts of marine plants, an extract of cucumber, an extract of clove, an extract of raspberry, an extract of balm, an extract of carrot, an extract of *Aesculus hippocastanum* L. (Horse Chestnut), an extract of peach, an extract of peach leaves, an extract of *Morus bombycis* Koidz., an extract of cornflower, an extract of *Hamamelis virginiana* L. (Virginean Witch Hazel), a Glycyrrhiza extract, an extract of *Ginkgo biloba* L. (Ginkgo), an extract of *Pyrola japonica* Klenze, an extract of Swertia herb, a capsicum tincture extract, and a cantharis extract. As the animal extracts, a placenta extract and collagen are used appropriately.

In the present invention, particularly, in cases where the water-soluble agent is crystalline at the normal temperature (25°C), the percutaneous absorption is capable of being enhanced markedly.

In the present invention, the application of the oil based composition for external use on skin need not necessarily be performed immediately after the preparation for external use on skin, which preparation contains the water-soluble agent, has been applied to the skin. For example, after the preparation for external use on skin, which preparation contains the water-soluble agent, has been applied to the skin and has then been dried slightly, the oil based composition for external use on skin may be applied from above the preparation for external use on skin, which preparation has been applied to the skin. Also, at least one kind of a different composition may be applied at a stage before the preparation for external use on skin, which preparation contains the water-soluble agent, is applied to the skin, or at a stage between the application of the preparation for external use on skin, which preparation contains the water-soluble agent, and the application of the oil based composition for external use on skin.

Further, at a stage after application of the preparation for external use on skin, which preparation contains the water-soluble agent, has been applied to the skin, and iontophoresis has then been performed, the application of the oil based composition for external use on skin may be performed.

The iontophoresis is the technique, wherein a comparatively weak electric current (e.g., approximately 10V, $0.5mA/cm^2$) is applied across the skin for several minutes to several hours, which has been brought into contact with a compound containing a water-soluble agent, a peptide type substance, or the like, to promote the percutaneous absorption of the water-soluble agent, the peptide type substance, or the like. For example, in cases where the agent is of the type negatively charged, a minus electrode may be brought into contact with the skin having been brought into contact with the composition (i.e., the minus pole may be utilized as the introduction pole), and the iontophoresis (cathodal IP) may thereby be performed. Also, in cases where the agent is of the type positively charged, a plus electrode may be brought into contact with the skin having been brought into contact with the composition (i.e., the plus pole may be utilized as the introduction pole), and the iontophoresis (anodal IP) may thereby be performed. In this manner, the percutaneous absorption of the agent is capable of being promoted. Therefore, in cases where the iontophoresis is performed, and the oil based composition for external use on skin in accordance with the present invention is then applied from above the water-soluble agent, the percutaneous absorption of the water-soluble agent is capable of being enhanced more efficiently.

The water-soluble agent of the type negatively charged is not limited to a specific agent. Examples of the water-soluble agents of the type negatively charged include ascorbic acid; ascorbic acid derivatives, such as ascorbic acid phosphoric acid, ascorbic acid-2-glucoside, ethylascorbic acid (vitamin C ethyl), e.g., 3-0-ethylascorbic acid or 2-0-ethylascorbic acid;and magnesium salts, calcium salts, and potassium salts of the agents enumerated above. Also, examples of the water-soluble agents of the type positively charged include basic amino acids, such as arginine.

As the technique, the apparatus, the voltage applying condition, and the like, for the iontophoresis, it is possible to employ the technique, the apparatus, the voltage applying condition, and the like, which are ordinarily employed. For example, as the electrodes, it is possible to utilize platinum electrodes, carbon electrodes, silver electrodes, silver chloride electrodes, and the like. Also, as the electricity applying technique, one of a wide variety of techniques, such as a direct type, a pulsed type, and a pulsed depolarization type, may be employed. Further, the electric current density is not limited to a specific value. The electric current density should preferably fall within the range of $0.001mA/cm^2$ to $0.5mA/cm^2$, shouldmore preferably fall within the range of $0.01mA/cm^2$ to $0.4mA/cm^2$, and should most preferably fall within the range of $0.05mA/cm^2$ to $0.3mA/cm^2$. Furthermore, no limitation is imposed upon the operation time. The operation time ordinarily fall within the range of 0.5 minute to 60 minutes per operation, and should preferably fall within the range of 1 minute to 30 minutes.

In the present invention, a patch sheet, in which a sheet type small electric cell has been embedded, may be used. In such cases, a weak current is capable of being applied to the skin more easily and more simply. For example, a weak current is capable of being applied to the skin easily by adhering a patch sheet, in which a 3V electric cell has been incorporated, to the skin via a hydrogel, which covers the electrodes. Change-over between the anodal IP and the cathodal IP is capable of being performed by changing the positions of the adhered electrodes. Also, the processing is capable of being ceased easily by removing the sheet from the skin. In such cases, the water-soluble agent may be blended in the hydrogel, which comes into contact with the skin.

In the present invention, no limitation is imposed upon the site of the skin, to which the preparation for external use on skin is applied. The site of the skin, to which the preparation for external use on skin is applied, embraces the skin of every area of the body surface, including the head skin.

The present invention will further be illustrated by the following examples.

Examples

1. Study of occlusivity

Oil based compositions for external use were prepared in accordance with recipes (Test Examples 1 to 13) listed in Table 1 below. With respect to each of the samples, the occlusivity and the percutaneous absorption enhancing effects were evaluated with the methods described below.

Evaluation of occlusivity:

The occlusivity of each of the compositions for external use were evaluated in accordance with the results of the measurement of the transepidermal water loss (TEWL). Specifically, as for each special panel of ten persons for each sample, the sample was applied (at a rate of 2.5mg/cm$^2$) to an internal site of the human forearm. At a stage one hour after the sample has been applied (at a rate of 2.5mg/cm$^2$) to the internal site of the human forearm, the transepidermal water loss (TEWL) was measured with a water loss meter (Tewameter TM210, supplied by Courage+Khazaka Co.). Thereafter, the occlusivity were calculated with the formula shown below and in accordance with the transepidermal water loss (TEWL) having thus been measured.

```
Occlusivity (%)
= (1-TEWL (with sample) / TEWL (without sample)) × 100
```

Evaluation of percutaneous absorption enhancing effects:

The percutaneous absorption enhancing effects of the composition for external use were evaluated by use of ascorbic acid phosphoric acid magnesium as the water-soluble agent. Specifically, as for each special panel of ten persons for each sample, an aqueous ascorbic acid phosphoric acid magnesium solution was applied (at a rate of 1$\mu$l/cm$^2$) to an inside site of the human forearm. After drying, the sample was applied (at a rate of 2mg/cm$^2$) from above. As for the control, the sample was not applied. After six hours had elapsed, the ascorbic acidphosphoric acidmagnesiumconcentration in the keratinwasmeasured. The relative value of the ascorbic acid phosphoric acid magnesium concentration in the cases of the application of each sample, which value was taken with respect to the measured value obtained for the control, was taken as the percutaneous absorption enhancing effects.
The results shown in Table 1 were obtained.
The compositons of Table 1 do not form part of the invention.

EP 1 839 647 B1

Table 1

| Recipe | Control | Test Ex. 1 | Test Ex. 2 | Test Ex. 3 | Test Ex. 4 | Test Ex. 5 | Test Ex. 6 |
|---|---|---|---|---|---|---|---|
| Petrolatum (non-polar hydrocarbon oil, semisolid) | | 100 | 80 | 65 | 50 | 30 | 15 |
| Micro-crystalline wax (non-polar hydrocarbon oil, solid) | | 0 | 0 | 0 | 10 | 20 | 20 |
| Liquid paraffin (non-polar hydrocarbon oil, liquid) | | 0 | 20 | 35 | 40 | 50 | 65 |
| Dimethylpolysiloxane (silicone oil, liquid) | | – | – | – | – | – | – |
| Trioctanoin (polar hydrocarbon oil, liquid) | | – | – | – | – | – | – |
| Evaluation | | | | | | | |
| Occlusivity (%) | – | 80% | 60% | 60% | 60% | 50% | 50% |
| Relative percutaneous absorption of APM | 1 | 3 | 2 | 2 | 2 | 1.5 | 1.5 |

Control: Without sample    APM: Ascorbic acid phosphoric acid magnesium

Table 1 - continued

| Recipe | Test Ex. 7 | Test Ex. 8 | Test Ex. 9 | Test Ex. 10 | Test Ex. 11 | Test Ex. 12 | Test Ex. 13 |
|---|---|---|---|---|---|---|---|
| Petrolatum (non-polar hydrocarbon oil, semisolid) | 0 | 15 | 0 | 85 | 85 | 65 | 75 |
| Micro-crystalline wax (non-polar hydrocarbon oil, solid) | 20 | 35 | 40 | 0 | 0 | 0 | 0 |
| Liquid paraffin (non-polar hydrocarbon oil, liquid) | 80 | 50 | 60 | 0 | 0 | 0 | 0 |
| Dimethylpolysiloxane (silicone oil, liquid) | - | - | - | 0 | 15 | 0 | 25 |
| Trioctanoin (polar hydrocarbon oil, liquid) | - | - | - | 15 | 0 | 35 | 0 |
| Evaluation | | | | | | | |
| Occlusivity (%) | 50% | 50% | 50% | 50% | 50% | 40% | 40% |
| Relative percutaneous absorption of APM | 1.5 | 1.5 | 1.5 | 1.5 | 1.5 | 1 | 1 |

Control: Without sample   APM: Ascorbic acid phosphoric acid magnesium

In cases where, after the water-soluble agent was applied to the skin, each of the oil based compositions for external use on skin (Test Examples 1 to 11) having the occlusivity of as high as at least 50% was applied from above the water-soluble agent having been applied to the skin, the percutaneous absorption of the water-soluble agent was capable of being enhanced markedly. In each of Test Examples 12 and 13, in which the occlusivity were lower than 50%, the percutaneous absorption was identical with the percutaneous absorption in the control, in which the oil based composition for external use on skin was not applied, and the percutaneous absorption enhancing effects were not capable of being obtained.

2. Study of improvement in usability

Since the usability was bad with the oily constituent alone due to low extensibility and the occurrence of a sticky feeling, the study was made to blend various particles for improving the usability such that the occlusivity of the oil based compositions for external use on skin might not be affected adversely.

Various kinds of the particles were blended with the oil constituents in accordance with the recipes shown in Table 2 below, and oil based compositions for external use on skin were prepared. With respect to each of the samples, the occlusivity and the percutaneous absorption enhancing effects were evaluated in the same manner as that described above. Also, the usability was evaluated in the manner described below.

Evaluation of usability:

The evaluation of the usability (the sticky feeling, the extensibility, and the powdery feeling) was made with sensory tests by a panel of specialists. Specifically, each of the samples was used by 10 females on the panel, and the usability was evaluated with the evaluation criteria shown below.

(Sticky feeling)

A: At least seven females of the panel answered that the sticky feeling was not given.
B: Three to six females of the panel answered that the sticky feeling was not given.
C: At most two females of the panel answered that the sticky feeling was not given.

(Extensibility)

A: At least seven females of the panel answered that the extensibility was high.
B: Three to six females of the panel answered that the extensibility was high.
C: At most two females of the panel answered that the extensibility was high.

(Powdery feeling)

A: At least seven females of the panel answered that the powdery feeling was not given.
B: Three to six females of the panel answered that the powdery feeling was not given.
C: At most two females of the panel answered that the powdery feeling was not given.

The results shown in Table 2 were obtained.

Table 2

| Recipe | Control | Ex. 1 | Ex. 2 | Ex. 3 | Ex. 4 | Ex. 5 | Ex. 6 (*9) |
|---|---|---|---|---|---|---|---|
| Petrolatum (*1) | | 80 | 25 | 25 | 25 | 20 | 80 |
| Micro-crystalline wax (*2) | | – | 15 | 15 | 15 | 12 | – |
| Liquid paraffin (*3) | | – | 40 | 40 | 40 | 32 | – |
| Decamethylcyclopentasiloxane (*4) | | – | – | – | – | 20 | – |
| Spherical silicone rubber particles (*5) | | 20 | – | – | – | – | – |
| Spherical silicone resin particles (*6) | | | 20 | – | – | – | – |
| Coated silicone rubber particles (*7) | | – | – | 20 | 18 | 16 | – |
| Zinc oxide (refractive index: 2.0) | | – | – | – | 2 | – | – |
| Talc (*8) | | – | – | – | – | – | 20 |
| Silica (spherical) | | – | – | – | – | – | – |
| Nylon (spherical) | | – | – | – | – | – | – |
| Evaluation | | | | | | | |
| Occlusivity (%) | | 70% | 60% | 70% | 70% | 60% | 60% |
| Relative percutaneous absorption of APM | 1 | 2.5 | 2 | 2.5 | 2.5 | 2 | 2 |
| Sticky feeling | | A | A | A | A | A | B |
| Extensibility | | B | B | B | B | A | B |
| Powdery feeling | | A | A | A | A | A | B |

Control: Without sample    APM: Ascorbic acid phosphoric acid magnesium

EP 1 839 647 B1

EP 1 839 647 B1

(*9)

Table 2 - continued

| Recipe | Ex. 7 (*9) | Ex. 8(*9) | Comp. Ex. 1 | Comp. Ex. 2 | Comp. Ex. 3 | Comp. Ex. 4 |
|---|---|---|---|---|---|---|
| Petrolatum (*1) | 75 | 75 | 100 | 30 | 50 | 30 |
| Micro-crystalline wax (*2) | - | - | - | 10 | - | - |
| Liquid paraffin (*3) | - | - | - | 60 | 49 | 5 |
| Decamethylcyclopentasiloxane (*4) | - | - | - | - | - | - |
| Spherical silicone rubber particles (*5) | - | - | - | - | 1 | 65 |
| Spherical silicone resin particles (*6) | - | - | - | - | - | - |
| Coated silicone rubber particles (*7) | - | - | - | - | - | - |
| Zinc oxide (refractive index: 2.0) | - | - | - | - | - | - |
| Talc (*8) | - | - | - | - | - | - |
| Silica (spherical) | 25 | - | - | - | - | - |
| Nylon (spherical) | - | 25 | - | - | - | - |
| Evaluation | | | | | | |
| Occlusivity (%) | 60% | 60% | 80% | 50% | 60% | 40% |
| Relative percutaneous absorption of APM | 2 | 2 | 3 | 1.5 | 2 | 1 |
| Sticky feeling | B | B | C | C | C | A |
| Extensibility | B | B | C | C | C | A |
| Powdery feeling | B | B | A | A | A | C |

Table 2 - continued

APM: Ascorbic acid phosphoric acid magnesium

Control: Without sample

*1: Non-polar hydrocarbon oil, semisolid

*2: Non-polar hydrocarbon oil, solid

*3: Non-polar hydrocarbon oil, liquid

*4: Silicone oil, non-polar oil, volatile

*5: Spherical, elastic, refractive index: 1.4

*6: Spherical, refractive index: 1.4

*7: Zinc oxide (refractive index: 2.0)-coated silicone resin-coated silicone rubber particles (spherical, elastic)

*8: Plate-shaped, refractive index: 1.57

*9: not part of the invention as claimed

In cases where the particles were blended in a proportion of at most 50% by mass, the sticky feeling and the extensibility were capable of being improved markedly, such that the occlusivity might not be affected adversely. Particularly, in cases where the spherical silicone type particles or the elastic silicone type particles were blended (in Examples 1 to 5), the coating and extension smoothness was good. Also, though not illustrated by the data, in cases where the particles having a refractive index of at least 1.6 or the particles having been coated with the particles having a refractive index of at least 1.6 were blended (in Examples 3 to 5), the protrusion and recesses of the skin were capable of being rendered imperceptible, and good color nonuniformity concealing effects were capable of being obtained. Further, in Example 5, in which the volatile liquid oil constituent was blended, the extensibility was capable of being improved even further. In Comparative Examples 1 and 2, in which the particles were not blended, and in Comparative Example 3, in which the blending proportion of the particles was lower than 5%, the sticky feeling occurred, and the extensibility was markedly bad. Also, in Comparative Example 4, in which the blending proportion of the particles was higher than 50% by mass, the occlusivity of as high as at least 50% were not capable of being obtained, and the percutaneous absorption of the water-soluble agent was not capable of being enhanced.

Further, though not illustrated by the results, experiments were made in the manner described below. Specifically, an aqueous ascorbic acid phosphoric acid magnesium solution was applied (at a rate of $1\mu l/cm^2$) to an inside site of the human forearm. Also, the iontophoresis from the side of the minus pole was performed. Thereafter, each of the oil based compositions for external use on skin in Examples described above was applied (at a rate of 2mg/cm$^2$). Insuchcases, the percutaneous absorption of ascorbic acid phosphoric acid magnesium was capable of being enhanced even further.

**Claims**

1.  An oil based composition for external use on skin for promoting percutaneous absorption of a water-soluble agent, the oil based composition containing:

    i) 50% by mass to 95% by mass of an oil constituent, which contains a solid or semisolid non-polar hydrocarbon oil in a proportion of at least 30% by mass with respect to the total quantity of the oil constituent, and
    ii) 5% by mass to 50% by mass of particles, which comprise at least one member selected from the group consisting of spherical silicone rubber particles, spherical silicone resin particles, silicone resin-coated silicone rubber particles, and zinc oxide-coated, silicone resin-coated silicone rubber particles,

    wherein the proportion of water and water-soluble constituents contained in the oil based composition is at most 1% by mass,
    wherein the oil based composition has occlusivity of at least 50%, and
    wherein the oil based composition is adapted for use in such a manner that it is applied onto a skin after a water-soluble agent-containing preparation for external use on skin; has been applied onto the skin.

2.  A composition as defined in Claim 1 wherein the oil constituent contains a volatile oil constituent in a proportion falling within the range of 5% by mass to 50% by mass with respect to the total quantity of the oil constituent.

3.  A cosmetic method, comprising the steps of:

    i) applying a cosmetic preparation, which contains a water-soluble agent, onto skin, and
    ii) applying an oil based composition for external use on skin onto the skin on which the cosmetic preparation has been applied,

    wherein the oil based composition contains:

    a) 50% by mass to 95% by mass of an oil constituent, which contains a solid or semisolid non-polar hydrocarbon oil in a proportion of at least 30% by mass with respect to the total quantity of the oil constituent, and
    b) 5% by mass to 50% by mass of particles, which comprise at least one member selected from the group consisting of spherical silicone rubber particles, spherical silicone resin particles, silicone resin-coated silicone rubber particles, and zinc oxide-coated, silicone resin-coated silicone rubber particles,

    wherein the proportion of water and water-soluble constituents contained in the oil based composition is at most 1% by mass, and
    wherein the oil based composition has occlusivity of at least 50%.

4.  A cosmetic method as defined in Claim 3, wherein the method further comprises the step of performing an iontophoresis on the skin at a stage between when the preparation for external use on skin or the cosmetic preparation, which contains the water-soluble agent, has been applied onto the skin and when the oil based composition for external use on skin is applied onto the cosmetic preparation, which has been applied onto the skin.

5.  A cosmetic method as defined in Claim 3 or 4 wherein the oil constituent contains a volatile oil constituent in a proportion falling within the range of 5% by mass to 50% by mass with respect to the total quantity of the oil constituent.

**Patentansprüche**

1.  Ölbasierte Zusammensetzung zur äußeren Anwendung auf Haut zur Förderung der perkutanen Absorption eines wasserlöslichen Agens, wobei die ölbasierte Zusammensetzung:

    i) 50 Masse-% bis 95 Masse-% eines Ölbestandteils, der ein festes oder halbfestes nicht-polares Kohlenwasserstofföl in einem Anteil von wenigstens 30 Masse-%, bezogen auf die Gesamtmenge des Ölbestandteils, enthält, und

ii) 5 Masse-% bis 50 Masse-% Teilchen, die wenigstens einen Bestandteil umfassen, der ausgewählt ist aus der Gruppe, bestehend aus sphärischen Silikonkautschuk-Teilchen, sphärischen Silikonharz-Teilchen, Silikonharz-beschichteten Silikonkautschuk-Teilchen und Zinkoxid-beschichteten, Silikonharz-beschichteten Silikonkautschuk-Teilchen,

enthält,

wobei der Anteil an Wasser und wasserlöslichen Bestandteilen, die in der ölbasierten Zusammensetzung enthalten sind, höchstens 1 Masse-% beträgt,

wobei die ölbasierte Zusammensetzung eine Okklusivität von wenigstens 50% aufweist und

wobei die ölbasierte Zusammensetzung in solcher Weise zur Anwendung angepasst ist, dass sie auf eine Haut aufgebracht wird, nachdem eine ein wasserlösliches Agens enthaltende Zubereitung zur äußeren Anwendung auf Haut auf die Haut aufgebracht worden ist.

2. Zusammensetzung nach Anspruch 1, wobei der Ölbestandteil einen flüchtigen Ölbestandteil in einem Anteil enthält, der in den Bereich von 5 Masse-% bis 50 Masse-%, bezogen auf die Gesamtmenge des Ölbestandteils, fällt.

3. Kosmetisches Verfahren, umfassend die Schritte:

i) Aufbringen einer kosmetischen Zubereitung, die ein wasserlösliches Agens enthält, auf Haut und
ii) Aufbringen einer ölbasierten Zusammensetzung zur äußeren Anwendung auf Haut auf die Haut, auf die die kosmetische Zubereitung aufgebracht worden ist,

wobei die ölbasierte Zusammensetzung:

a) 50 Masse-% bis 95 Masse-% eines Ölbestandteils, der ein festes oder halbfestes nicht-polares Kohlenwasserstofföl in einem Anteil von wenigstens 30 Masse-%, bezogen auf die Gesamtmenge des Ölbestandteils, enthält, und

b) 5 Masse-% bis 50 Masse-% Teilchen, die wenigstens einen Bestandteil umfassen, der ausgewählt ist aus der Gruppe, bestehend aus sphärischen Silikonkautschuk-Teilchen, sphärischen Silikonharz-Teilchen, Silikonharz-beschichteten Silikonkautschuk-Teilchen und Zinkoxid-beschichteten, Silikonharz-beschichteten Silikonkautschuk-Teilchen,

enthält,

wobei der Anteil an Wasser und wasserlöslichen Bestandteilen, die in der ölbasierten Zusammensetzung enthalten sind, höchstens 1 Masse-% beträgt und

wobei die ölbasierte Zusammensetzung eine Okklusivität von wenigstens 50% aufweist.

4. Kosmetisches Verfahren nach Anspruch 3, wobei das Verfahren weiter den Schritt der Durchführung einer Iontophorese auf der Haut in einem Stadium zwischen dem Zeitpunkt, wenn die Zubereitung für äußere Anwendung auf Haut oder die kosmetische Zubereitung, die das wasserlösliche Agens enthält, auf die Haut aufgebracht worden ist, und diejenigen, wenn die ölbasierte Zusammensetzung für äußere Anwendung auf Haut auf die kosmetische Zubereitung, die auf die Haut aufgebracht worden ist, aufgebracht ist, umfasst.

5. Kosmetisches Verfahren nach Anspruch 3 oder 4, wobei der Ölbestandteil einen flüchtigen Ölbestandteil in einem Anteil enthält, der in den Bereich von 5 Masse-% bis 50 Masse-%, bezogen auf die Gesamtmenge des Ölbestandteils, fällt.

## Revendications

1. Composition à base d'huile pour usage externe sur la peau afin de favoriser l'absorption percutanée d'un agent soluble dans l'eau, la composition à base d'huile contenant :

i) entre 50% en masse et 95% en masse d'un constituant huileux, qui contient une huile d'hydrocarbure non polaire solide ou semi-solide dans une proportion d'au moins 30% en masse par rapport à la quantité totale du constituant huileux, et

ii) entre 5% en masse et 50% en masse de particules, qui comprennent au moins un élément choisi dans le groupe constitué de particules sphériques de caoutchouc de silicone, de particules sphériques de résine de silicone, de particules de caoutchouc de silicone revêtues de résine de silicone, et de particules de caoutchouc de silicone revêtues de résine de silicone et d'oxyde de zinc,

où la proportion d'eau et de constituants solubles dans l'eau contenus dans la composition à base d'huile atteint au maximum 1% en masse,
où la composition à base d'huile a une occlusivité d'au moins 50%, et
où la composition à base d'huile est adaptée pour être utilisée de sorte à être appliquée sur une peau après application sur la peau d'une préparation contenant un agent soluble dans l'eau pour usage externe sur la peau.

2. Composition telle que définie dans la revendication 1 dans laquelle le constituant huileux contient un constituant huileux volatile dans une proportion dans la plage de 5% en masse jusqu'à 50% en masse par rapport à la quantité totale du constituant huileux.

3. Procédé cosmétique, comprenant les étapes qui consistent :

i) à appliquer une préparation cosmétique, qui contient un agent soluble dans l'eau, sur la peau, et
ii) à appliquer une composition à base d'huile pour usage externe sur la peau sur laquelle la préparation cosmétique a été appliquée,

où la composition à base d'huile contient :

a) entre 50% en masse et 95% en masse d'un constituant huileux, qui contient une huile d'hydrocarbure non polaire solide ou semi-solide dans une proportion d'au moins 30% en masse par rapport à la quantité totale du constituant huileux, et
b) entre 5% en masse et 50% en masse de particules, qui comprennent au moins un élément choisi dans le groupe constitué de particules sphériques de caoutchouc de silicone, de particules sphériques de résine de silicone, de particules de caoutchouc de silicone revêtues de résine de silicone, et particules de caoutchouc de silicone revêtues de résine de silicone et d'oxyde de zinc,

où la proportion d'eau et des constituants solubles dans l'eau contenus dans la composition à base d'huile atteint au maximum 1% en masse, et
où la composition à base d'huile a une occlusivité d'au moins 50%.

4. Procédé cosmétique tel que défini dans la revendication 3, dans lequel le procédé comprend en outre l'étape consistant à exécuter une iontophorèse sur la peau entre le moment où la préparation pour usage externe sur la peau ou la préparation cosmétique, qui contient l'agent soluble dans l'eau, a été appliquée sur la peau et le moment où la composition à base d'huile pour usage externe sur la peau est appliquée sur la préparation cosmétique, qui a été appliquée sur la peau.

5. Procédé cosmétique tel que défini dans la revendication 3 ou 4 dans lequel le constituant huileux contient un constituant huileux volatile dans une proportion dans la plage de 5% en masse jusqu'à 50% en masse par rapport à la quantité totale du constituant huileux.

PRIOR ART

WATER-SOLUBLE AGENT

EPIDERMIS

PREPARATION FOR EXTERNAL USE FOR SKINS

**FIG.1A**

OIL BASED COMPOSITION FOR EXTERNAL USE FOR SKINS, WHICH COMPOSITION HAS BEEN APPLIED WITH THE POST-PROCESSING

HYDRATE LAYER

POST-PROCESSING

WATER-SOLUBLE AGENT

EPIDERMIS

PREPARATION FOR EXTERNAL USE FOR SKINS

**FIG.1B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5747066 A
- JP 9157129 A
- JP 5229927 A
- JP 2000178125 A
- JP 2003026608 A
- JP 6316516 A
- JP 9012429 A
- US 2004197281 A1
- WO 02060502 A2
- US 5326566 A
- US 2003228335 A1